# EUROPEAN PATENT APPLICATION

(11) **EP 3 756 669 A1**
(43) Date of publication of application: **30.12.2020**
(21) Application number: 20168406.5
(22) Date of filing: 06.01.2014
(51) Int. Cl.: A61K 31/4745, A61K 9/06, A61P 35/00, A61P 17/00, A61P 35/02, A61P 37/04, A61P 43/00, A61K 9/00

(54) **COMPOSITIONS FOR USE FOR TREATING CUTANEOUS T CELL LYMPHOMA**

(30) Priority: 07.01.2013 US 201361749739 P
(62) Document number(s) of the earlier application(s) in accordance with Art. 76 EPC: 14735313.0 / 2 941 233
(71) Applicant: The Trustees of the University of Pennsylvania, Philadelphia PA 19104-6283 (US)
(72) Inventor: ROOK, Alain H., Wynnewood, PA Pennsylvania 19096 (US); GELFAND, Joel M., Penn Valley, PA Pennsylvania 19072 (US); WYSOCKA, Maria M., Drexel Hill, PA Pennsylvania 19026 (US); BENOIT, Bernice M., Prospect Park, PA Pennsylvania 19076 (US); TROXEL, Andrea Beth, Bala Cynwyd, PA Pennsylvania 19004 (US)
(74) Representative: Grünecker Patent- und Rechtsanwälte PartG mbB

(57) **Abstract**

The present invention provides methods for treating a subject with cutaneous T cell lymphoma (CTCL). In one embodiment, a subject is administered an effective amount effective of an IRM compound wherein at least one symptom or clinical sign of CTCL is ameliorated. The present invention further includes a method of increasing a cell-mediated immune response of a cell population that includes cells affected by CTCL. In one embodiment, an effective amount of the cell population is contacted with an IRM compound wherein at least one cell-mediated immune activity of the cell population is increased.

## Description

### CROSS-REFERENCE TO RELATED APPLICATIONS

The present application claims priority under 35 U.S.C. § 119(e) to U.S. Provisional Application No. 61/749,739, filed January 7, 2013, which application is hereby incorporated by reference herein in its entirety.

### STATEMENT REGARDING FEDERALLY SPONSORED RESEARCH OR DEVELOPMENT

This invention was made with government support under grants number 5R01 CA122569-4 and R01 FD004092 awarded by the National Institutes of Health. The government has certain rights in the invention.

### BACKGROUND OF THE INVENTION

Cutaneous T-cell lymphoma (CTCL) is a relatively rare disease, with an annual incidence of about 0.29 cases per 100,000 persons in the United States. Even though CTCL is reported to be about half as common in Eastern Europe, this discrepancy may be attributed to a differing physician awareness of the disease rather than a true difference in occurrence. In the United States, there are about 1,500 new cases identified each year, and about 100-200 deaths yearly. CTCL is usually seen in older adults (the median age at diagnosis is 55-60 years), and strikes twice as many men as women. The average life expectancy at diagnosis is 7-10 years, even without treatment.

CTCL is an indolent (low grade) cancer of the white blood cells that primarily affects the skin and only secondarily affects other sites. This disease involves the uncontrollable proliferation of T lymphocytes known as helper T (TH) cells. The proliferation of helper T cells results in the penetration, or infiltration, of these abnormal cells into the dermal and epidermal layers of the skin. The skin may react with itchy, slightly scaling lesions, although the sites of greatest infiltration do not necessarily correspond to the sites of the lesions. The lesions are most often located on the trunk, but can be present on any part of the body. In the most common course of the disease, also known as mycosis fungoides (MF), the patchy lesions progress to palpable plaques that are deeper red and have more defined edges. Eventually, skin tumors may develop. Finally, the cancer may progress to extracutanous involvement, often in the lymph nodes or the viscera. In rare cases, affected individuals may develop Sezary syndrome (SS), a leukemic variant of MF.

The proliferative T lymphocytes of CTCL are characterized by the phenotype CD4+/CD45RO+/CLA+/CCR4+. MF and SS differ in the involvement of the peripheral blood. MF typically appears without overt involvement of the peripheral blood by circulating malignant T cells, whereas SS typically includes malignant T cells disseminated into the blood stream. Involvement of the peripheral blood is typically associated with a decrease in cell-mediated immunity including a decrease in the production of TH1-type cytokines such as, for example, IFN-γ and IL-2, and increased production of TH2-type cytokines such as, for example, IL-4 and IL-5.

CTCL patients, particularly SS patients, are deficient in IL-12 production resulting, at least in part, from decreased numbers of myeloid dendritic cells, which are important IL-12 producers. IL-12 stimulates proliferation of NK cells and T cells, increases cytolytic activity of NK cells, and stimulates IFN-γ production, which in turn enhances production of IL-12 by DCs and monocytes.

Exogenous administration of TH1-type cytokines produces measurable clinical responses in treated patients. For example, administration of IFN-α, IFN-γ, and/or IL-12 have been used in such therapies, but identification of effective therapeutic agents with a low occurrence of side effects and an ability to stimulate multiple components of the immune system continues.

There has been a major effort in recent years to discover new drug compounds that act by stimulating certain key aspects of the immune system, as well as by suppressing certain other aspects (see, e.g., U.S. Patent Nos. 6,039,969 and 6,200,592). These compounds, referred to herein as immune response modifiers (IRMs), appear to act through basic immune system mechanisms known as Toll-like receptors (TLRs) leading to cytokine biosynthesis, induction of co-stimulatory molecules, and increased antigen-presenting capacity.

They may be useful for treating a wide variety of diseases and conditions. For example, certain IRMs may be useful for treating viral diseases (e.g., human papilloma virus, hepatitis, herpes), neoplasias (e.g., basal cell carcinoma, squamous cell carcinoma, actinic keratosis, melanoma), and TH2-mediated diseases (e.g., asthma, allergic rhinitis, .atopic dermatitis), auto-immune diseases (e.g., multiple sclerosis), and are also useful as vaccine adjuvants.

Many IRM compounds are small organic molecule imidazoquinoline amine derivatives (see, e.g., U.S. Patent No. 4,689,338), but a number of other compound classes are known as well (see, e.g., U.S. Patent Nos. 5,446,153; 6,194,425; and 6,110,929; and International Publication No. WO 2005/0791 95) and more are still being discovered. Other IRMs have higher molecular weights, such as oligonucleotides, including CpGs (see, e.g., U.S. Patent No. 6,194,388).

There is still a need in the art for novel and improved compositions that may be used to treat refractory diseases or disorders such as CTCL. The present invention addresses this need.

### BRIEF SUMMARY OF THE INVENTION

In one aspect, the invention includes a method of treating or ameliorating cutaneous T-cell lymphoma (CTCL) in a subject in need thereof. In certain non-limiting embodiments, the method comprises administering to the subject topically, transdermally, intradermally or intralesionally a therapeutically effective amount of a pharmaceutical composition comprising an immune response modifier (IRM) compound, whereby the CTCL in the subject is treated or ameliorated.

In another aspect, the invention includes a method of increasing a cell-mediated immune response in a subject suffering from CTCL. In certain non-limiting embodiments, the method comprises administering to the subject topically, transdermally, intradermally, or intralesionally a therapeutically effective amount of a pharmaceutical composition comprising an IRM compound, whereby the cell-mediated immune response in the subject is increased.

In various embodiments of any of the above aspects or any aspect of the invention delineated herein, the administration of the composition to the subject is topical. In certain embodiments of the invention, the IRM compound comprises 4-amino-**α,α**-dimethyl-2-ethoxymethyl-1H-imidazo[4,5-c]quinolin-1-ethanol or a pharmaceutically acceptable salt thereof. In other embodiments of the invention, the composition comprises a gel. In yet other embodiments of the invention, the composition comprises from about 0.01% (w/w) IRM to about 0.5% (w/w) IRM. In yet other embodiments of the invention, the composition comprises from about 0.03% (w/w) IRM to about 0.06% (w/w) IRM.

In various embodiments of any of the above aspects or any aspect of the invention delineated herein, the composition is applied to at least one CTCL lesion of the subject. In certain embodiments of the invention, the administration results in at least partial clearing of the at least one CTCL lesion to which the composition was applied. In other embodiments of the invention, the administration results in at least partial clearing of at least one CTCL lesion to which the composition was not applied. In yet other embodiments of the invention, the administration results in 50% or greater clearing of the total CTCL lesions in the subject. In yet other embodiments of the invention, the amount of the IRM administered to the subject is from about 1 µg/kg to about 10 mg/kg. In yet other embodiments of the invention, the amount of the IRM administered to the subject is from about 100 ng/lesion to about 1 mg/lesion. In yet other embodiments of the invention, the composition is administered to the subject at a frequency of at least once per day, at least once per week, or at least once per month. In yet other embodiments of the invention, the composition is administered to the subject repeatedly over a duration of at least one day, at least one week, at least one month, or at least one year.

In various embodiments of any of the above aspects or any aspect of the invention delineated herein, the administration activates a systemic cell-mediated antitumor immune response in the subject. In certain embodiments of the invention, the administration induces infiltration of activated NK cells or activated T-cells in at least one lesion in the subject. In other embodiments of the invention, the administration results in an increase level of granzyme or IFN **α** in at least one lesion in the subject. In yet other embodiments of the invention, the administration activates circulating myeloid dendritic cells or circulating NK cells in the blood of the subject. In yet other embodiments of the invention, the activated circulating myeloid dendritic cells have increased CD80 expression.

In various embodiments of any of the above aspects or any aspect of the invention delineated herein, the composition is administered to the subject during a first treatment period and during a second treatment period, and wherein the first treatment period and the second treatment period are separated by a non-treatment period. In certain embodiments of the invention, the composition is administered to the subject during the first treatment period at a frequency of at least once per day, at least once per week, or at least once per month. In other embodiments of the invention, the gel is administered to the subject during the second treatment period at a frequency of at least once per day, at least once per week, or at least once per month. In yet other embodiments of the invention, the first treatment period is at least about two weeks, at least about three weeks, at least about four weeks, at least about five weeks, at least about six weeks, at least about seven weeks, or at least about eight weeks. In yet other embodiments of the invention, the second treatment period is at least about two weeks, at least about three weeks, at least about four weeks, at least about five weeks, at least about six weeks, at least about seven weeks, or at least about eight weeks. In yet other embodiments of the invention, the non-treatment period separating the first treatment period and the second treatment period is at least about one week, at least about two weeks, at least about three weeks, or at least about four weeks. In yet other embodiments of the invention, the subject is a mammal. In yet other embodiments of the invention, the mammal is human.

### BRIEF DESCRIPTION OF THE DRAWINGS

The following detailed description of preferred embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities of the embodiments shown in the drawings.

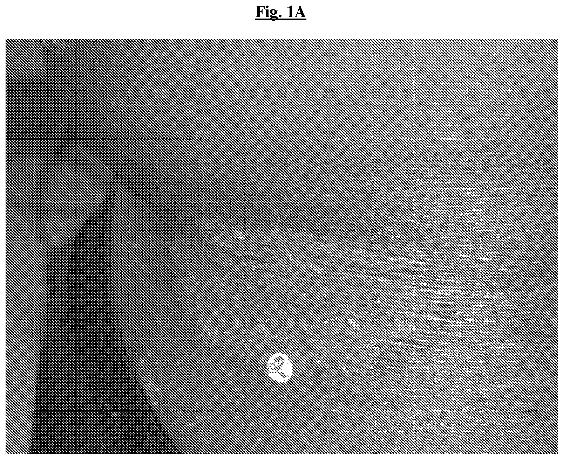

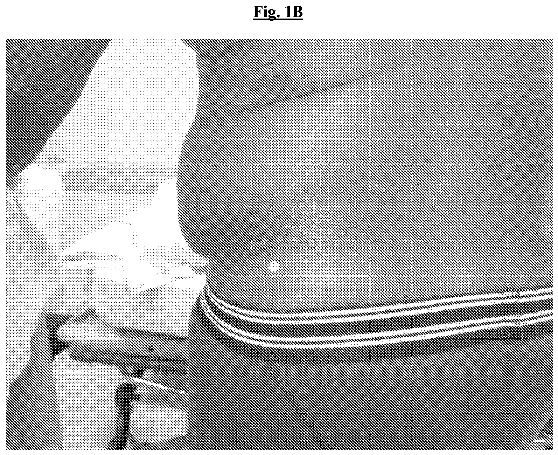

Figure 1, comprising Figures 1A-1B, comprises photographs of a patient enrolled in a clinical trial recited in the present application. Figure 1A illustrates the skin lesion before the application of resiquimod, and Figure 1B illustrates the skin lesion at the end of week 8 of treatment. These results demonstrate that resiquimod clears the treated lesions and induces resolution of distant lesions.

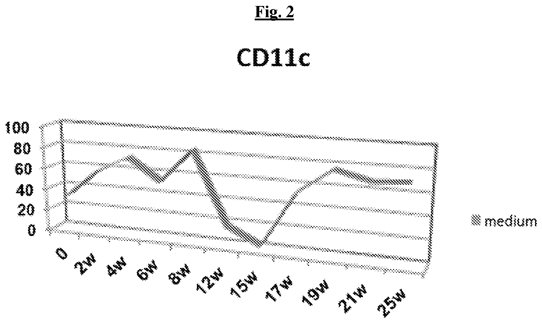

Figure 2 is a graph illustrating the percentage of CD80-positive myeloid dendritic cells in the peripheral blood of patients enrolled in the study. These results indicate that CD80 expression was activated in these cells during the initial eight weeks of therapy and again after the four-week treatment hiatus ending week 12.

### DETAILED DESCRIPTION OF THE INVENTION

The present invention includes a method of treating cutaneous T cell lymphoma (CTCL) in a subject. Patients with advanced CTCL have a significantly impaired ability to generate a cell-mediated immune response, and this impairment makes it difficult for the patient's immune system to control and contain CTCL. In a non-limiting embodiment, the invention uses immune response modifier (IRM) compounds to stimulate immune responses by other, still responsive immune cell populations to help control and contain the CTCL.

The present invention is based in part on the unexpected discovery that a formulation comprising resiquimod (4-amino-**α,α**-dimethyl-2-ethoxymethyl-1H-imidazo[4,5-c]quinolin-1-ethanol or a salt thereof) is particularly advantageous for treating CTCL because it provides a local pro-inflammatory effect on treated target lesions, while distant lesions also respond due to systemic immune activation.

As described herein, treated target lesions exhibit a marked intralesional influx of activated cytotoxic T-cells and NK cells manifesting increased expression of granzyme and IFN-γ. The disclosure further provides evidence for systemic immune augmentation, including the progressive activation of circulating myeloid dendritic cells and NK cells in the blood. In particular embodiments, the formulations of the present invention induce high clinical response rates of both locally treated target lesion, as well as distant lesions, and possess the ability to significantly boost systemic cellular immunity. The disclosures herein are relevant to the treatment of not only CTCL, but other skin cancers as well.

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described.

As used herein, each of the following terms has the meaning associated with it in this section.
The articles "a" and "an" are used herein to refer to one or to more than one (*i.e.*, to at least one) of the grammatical object of the article. By way of example, "an element" means one element or more than one element.
"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.
As used herein, the term "IRM" refers to immune response modifier.
"Agonist" as used herein refers to a compound that can combine with a receptor (e.g., a TLR) to induce a cellular activity. An agonist may be a ligand that directly binds to the receptor. Alternatively, an agonist may combine with a receptor indirectly by, for example, (a) forming a complex with another molecule that directly binds to the receptor, or (b) otherwise results in the modification of another compound so that the other compound directly binds to the receptor. An agonist may be referred to as an agonist of a particular TLR (e.g., a TLR6 agonist) or a particular combination of TLRs (e.g., a TLR 7/8 agonist-an agonist of both TLR7 and TLR8).
"Cell-mediated immune activity" as used herein refers to a biological activity considered part of a cell-mediated immune response such as, for example, an increase in the production of at least one TH1 cytokine.
"Immune cell" as used herein refers to cell of the immune system, i.e., a cell directly or indirectly involved in the generation or maintenance of an immune response, whether the immune response is innate, acquired, humoral, or cell-mediated.
"Sign" or "clinical sign" as used herein refers to an objective physical finding relating to a particular condition capable of being found by one other than the patient.
"Symptom" as used herein refers to any subjective evidence of disease or of a patient's condition.
"Clearing" as applied to a lesion refers to the reduction in size, number and/or gravity of one or more lesions in a subject. Thus, the term "clearing" may be applicable to a single lesion, a subset of the total lesion, or the total lesions of a subject. In one embodiment, the clearing takes place in at least one lesion to which the composition of the invention is administered. In another embodiment, the clearing takes place in at least one lesion to which the composition of the invention is not administered.
The term "abnormal," when used in the context of organisms, tissues, cells or components thereof, refers to those organisms, tissues, cells or components thereof that differ in at least one observable or detectable characteristic (e.g., age, treatment, time of day, etc.) from those organisms, tissues, cells or components thereof that display the "normal" (expected) respective characteristic. Characteristics that are normal or expected for one cell or tissue type might be abnormal for a different cell or tissue type.
A "disease" as used herein is a state of health of an animal wherein the animal cannot maintain homeostasis, and wherein if the disease is not ameliorated then the animal's health continues to deteriorate.
In contrast, as used herein a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.
As used herein, a disease or disorder is "alleviated" or "treated" if the severity of a symptom of the disease or disorder, the frequency with which such a symptom is experienced by a patient, or both, is reduced.
"Ameliorate" as used herein refers to any reduction in the extent, severity, frequency, and/or likelihood of a symptom or clinical sign characteristic of a particular condition.
As used herein, a "therapeutic" treatment is a treatment administered to a subject who exhibits signs of pathology, for the purpose of diminishing or eliminating those signs.
As used herein, the term "treatment" or "treating" is defined as the application or administration of a therapeutic agent, i.e., a compound useful within the invention (alone or in combination with another pharmaceutical agent), to a patient, or application or administration of a therapeutic agent to an isolated tissue or cell line from a patient (e.g., for diagnosis or ex vivo applications), who has a disease or disorder, a symptom of a disease or disorder or the potential to develop a disease or disorder, with the purpose to cure, heal, alleviate, relieve, alter, remedy, ameliorate, improve or affect the disease or disorder, the symptoms of the disease or disorder, or the potential to develop the disease or disorder. Such treatments may be specifically tailored or modified, based on knowledge obtained from the field of pharmacogenomics.
As used herein, the term "prevent" or "prevention" means no disorder or disease development if none had occurred, or no further disorder or disease development if there had already been development of the disorder or disease. Also considered is the ability of one to prevent some or all of the symptoms associated with the disorder or disease.
As used herein, the term "patient," "individual" or "subject" refers to a human or a non-human mammal. Non-human mammals include, for example, livestock and pets, such as ovine, bovine, porcine, canine, feline and murine mammals. Preferably, the patient, individual or subject is human.
As used herein, the terms "effective amount," "pharmaceutically effective amount" and "therapeutically effective amount" refer to a nontoxic but sufficient amount of an agent to provide the desired biological result. That result may be reduction and/or alleviation of the signs, symptoms, or causes of a disease, or any other desired alteration of a biological system. An appropriate therapeutic amount in any individual case may be determined by one of ordinary skill in the art using routine experimentation.
As used herein, the term "pharmaceutically acceptable" refers to a material, such as a carrier or diluent, which does not abrogate the biological activity or properties of the compound, and is relatively non-toxic, i.e., the material may be administered to an individual without causing undesirable biological effects or interacting in a deleterious manner with any of the components of the composition in which it is contained.
As used herein, the language "pharmaceutically acceptable salt" refers to a salt of the administered compound prepared from pharmaceutically acceptable non-toxic acids and bases, including inorganic acids, inorganic bases, organic acids, inorganic bases, solvates, hydrates, and clathrates thereof. Suitable pharmaceutically acceptable acid addition salts may be prepared from an inorganic acid or from an organic acid. Examples of inorganic acids include sulfate, hydrogen sulfate, hydrochloric, hydrobromic, hydriodic, nitric, carbonic, sulfuric, and phosphoric acids (including hydrogen phosphate and dihydrogen phosphate). Appropriate organic acids may be selected from aliphatic, cycloaliphatic, aromatic, araliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, fumaric, pyruvic, aspartic, glutamic, benzoic, anthranilic, 4-hydroxybenzoic, phenylacetic, mandelic, embonic (pamoic), methanesulfonic, ethanesulfonic, benzenesulfonic, pantothenic, trifluoromethanesulfonic, 2-hydroxyethanesulfonic, p-toluenesulfonic, sulfanilic, cyclohexylaminosulfonic, stearic, alginic, **β**-hydroxybutyric, salicylic, galactaric and galacturonic acid. Suitable pharmaceutically acceptable base addition salts of compounds of the invention include, for example, metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, N,N'-dibenzylethylene-diamine, chloroprocaine, choline, diethanolamine, ethylenediamine, meglumine (N-methylglucamine) and procaine. All of these salts may be prepared from the corresponding compound by reacting, for example, the appropriate acid or base with the compound.
As used herein, the term "composition" or "pharmaceutical composition" refers to a mixture of at least one compound useful within the invention with a pharmaceutically acceptable carrier. The pharmaceutical composition facilitates administration of the compound to a patient. Multiple techniques of administering a compound exist in the art including, but not limited to, intravenous, oral, aerosol, inhalational, rectal, vaginal, transdermal, intranasal, buccal, sublingual, parenteral, intrathecal, intragastrical, ophthalmic, pulmonary and topical administration.
As used herein, the term "pharmaceutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the invention within or to the patient such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the invention, and not injurious to the patient. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations. As used herein, "pharmaceutically acceptable carrier" also includes any and all coatings, antibacterial and antifungal agents, and absorption delaying agents, and the like that are compatible with the activity of the compound useful within the invention, and are physiologically acceptable to the patient. Supplementary active compounds may also be incorporated into the compositions. The "pharmaceutically acceptable carrier" may further include a pharmaceutically acceptable salt of the compound useful within the invention. Other additional ingredients that may be included in the pharmaceutical compositions used in the practice of the invention are known in the art and described, for example in Remington's Pharmaceutical Sciences (Genaro, Ed., Mack Publishing Co., 1985, Easton, PA), which is incorporated herein by reference.
As used herein, an "effective amount" of a delivery vehicle is that amount sufficient to effectively bind or deliver a compound.
As used herein, the term "potency" refers to the dose needed to produce half the maximal response (ED₅₀).
As used herein, the term "efficacy" refers to the maximal effect (Eₘₐₓ) achieved within an assay.
Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Description

CTCL is an indolent (low grade) cancer of the white blood cells that primarily affects the skin and only secondarily affects other sites of the subject. This disease involves the uncontrollable proliferation of T lymphocytes known as helper T (TH) cells. The proliferation of helper T cells results in the penetration, or infiltration, of these abnormal cells into the dermal and epidermal layers of the skin.

Immune response modifiers ("IRMs") include compounds that possess immunomodulating activity, including, but not limited to, antiviral and antitumor activity. Certain IRMs modulate the production and secretion of cytokines. For example, certain IRM compounds induce the production and secretion of cytokines such as, for example, Type I interferons, TNF-**α**, IL-1, IL-6, IL-8, IL-10, IL-12, MIP-1, and/or MCP-1. As another example, certain IRM compounds can inhibit production and secretion of certain TH2 cytokines, such as IL-4 and IL-5. Additionally, some IRM compounds are reported to suppress IL-1 and TNF (see U.S. Patent No. 6,518,265).

Certain IRMs are small organic molecules (e.g., molecular weight under about 1,000 Daltons, preferably under about 500 Daltons) such as those disclosed in, for example, U.S. Patent Nos. 4,689,338; 4,929,624; 5,266,575; 5,268,376; 5,346,905; 5,352,784; 5,389,640; 5,446,153; 5,482,936; 5,756,747; 6,110,929; 6,194,425; 6,331,539; 6,376,669; 6,451,810; 6,525,064; 6,541,485; 6,545,016; 6,545,017; 6,573,273; 6,656,938; 6,660,735; 6,660,747; 6,664,260; 6,664,264; 6,664,265; 6,667,312; 6,670,372; 6,677,347; 6,677,348; 6,677,349; 6,683,088; 6,756,382; 6,797,718; 6,818,650; and 7,7091,214; U.S. Patent Publication Nos. 2004/0091491; 2004/0176367; and 2006/0100229; and International Publication Nos. WO 2005/18551, WO 2005/18556, WO 2005/20999, WO 2005/032484, WO 2005/048933, WO 2005/048945, WO 2005/051317, WO 2005/051324, WO 2005/066169, WO 2005/066170, WO 2005/066172, WO 2005/076783, WO 2005/079195, WO 2005/094531, WO 2005/123079, WO 2005/123080, WO 2006/009826, WO 2006/009832, WO 2006/026760, WO 2006/028451, WO 2006/028545, WO 2006/028962, WO 2006/029115, WO 2006/038923, WO 2006/065280, WO 2006/074003, WO 2006/083440, WO 2006/086449, WO 2006/091394, WO 2006/086633, WO 2006/086634, WO 2006/091567, WO 2006/091568, WO 2006/091647, WO 2006/093514, and WO 2006/098852.

Additional examples of small molecule IRMs include certain purine derivatives (such as those described in U.S. Patent Nos. 6,376,501, and 6,028,076), certain imidazoquinoline amide derivatives (such as those described in U.S. Patent No. 6,069,149), certain imidazopyridine derivatives (such as those described in U.S. Patent No. 6,518,265), certain benzimidazole derivatives (such as those described in U.S. Patent 6,387,938), certain derivatives of a 4-aminopyrimidine fused to a five membered nitrogen containing heterocyclic ring (such as adenine derivatives described in U. S. Patent Nos. 6,376,501; 6,028,076 and 6,329,381; and in WO 02/08905), and certain 3-**β**-D-riboruranosylthiazolo[4,5-d]pyrimidine derivatives (such as those described in U.S. Publication No. 2003/0199461), and certain small molecule immuno-potentiator compounds such as those described, for example, in U.S. Patent Publication No. 2005/0136065.

In some embodiments of the present invention, the IRM compound may be a small molecule immune response modifier (e.g., molecular weight of less than about 1,000 Daltons). In preferred embodiments, the IRM compound comprises 4-amino-**α,α**-dimethyl-2-ethoxymethyl-1H-imidazo[4,5-c]quinolin-1-ethanol (i.e., resiquimod) or a salt thereof.

Unless otherwise indicated, reference to a compound can include the compound in any pharmaceutically acceptable form, including any isomer (e.g., diastereomer or enantiomer), salt, solvate, polymorph, and the like. In particular, if a compound is optically active, reference to the compound can include each of the compound's enantiomers as well as racemic mixtures of the enantiomers.

In some embodiments of the present invention, the IRM compound may be an agonist of at least one TLR such as, for example, at least one of TLR7 or TLR8. The IRM may also in some cases be an agonist of TLR9. In some embodiments, the IRM compound may be an agonist of at least one of TLR7 and TLR8 such as, for example, a TLR7/8 agonist, a TLR8-selective agonist, or a TLR7-selective agonist.

As used herein, the term "TLR8-selective agonist" refers to any compound that acts as an agonist of TLR8, but does not act as an agonist of TLR7.

As used herein, the term "TLR7-selective agonist" refers to a compound that acts as an agonist of TLR7, but does not act as an agonist of TLR8.

As used herein, the term "TLR7/8 agonist" refers to a compound that acts as an agonist of both TLR7 and TLR8.

A TLR8-selective agonist or a TLR7-selective agonist may act as an agonist for the indicated TLR and one or more of TLR1, TLR2, TLR3, TLR4, TLR5, TLR6, TLR9, or TLR10. Accordingly, while "TLR8-selective agonist" may refer to a compound that acts as an agonist for TLR8 and for no other TLR, it may alternatively refer to a compound that acts as an agonist of TLR8 and, for example, TLR6. Similarly, "TLR7-selective agonist" may refer to a compound that acts as an agonist for TLR7 and for no other TLR, but it may alternatively refer to a compound that acts as an agonist of TLR7 and, for example, TLR6.

The TLR agonism for a particular compound may be assessed in any suitable manner. For example, assays and recombinant cell lines suitable for detecting TLR agonism of test compounds are described, for example, in U.S. Patent Publication Nos. US 2004/0014779, US 2004/0132079, US 2004/0162309, US 2004/0171086, US 2004/0191833, and US 2004/0197865.

Regardless of the particular assay employed, a compound may be identified as an agonist of a particular TLR if performing the assay with a compound results in at least a threshold increase of some biological activity mediated by the particular TLR. Conversely, a compound may be identified as not acting as an agonist of a specified TLR if, when used to perform an assay designed to detect biological activity mediated by the specified TLR, the compound fails to elicit a threshold increase in the biological activity. Unless otherwise indicated, an increase in biological activity refers to an increase in the same biological activity over that observed in an appropriate control. An assay may or may not be performed in conjunction with the appropriate control. With experience, one skilled in the art may develop sufficient familiarity with a particular assay (e.g., the range of values observed in an appropriate control under specific assay conditions) that performing a control may not always be necessary to determine the TLR agonism of a compound in a particular assay.

The precise threshold increase of TLR-mediated biological activity for determining whether a particular compound is or is not an agonist of a particular TLR in a given assay may vary according to factors known in the art, including, but not limited, to the biological activity observed as the endpoint of the assay, the method used to measure or detect the endpoint of the assay, the signal-to-noise ratio of the assay, the precision of the assay, and whether the same assay is being used to determine the agonism of a compound for more than one TLR. Accordingly it is not practical to set forth generally the threshold increase of TLR-mediated biological activity required to identify a compound as being an agonist or a non-agonist of a particular TLR for all possible assays. Those of ordinary skill in the art, however, can readily determine the appropriate threshold with due consideration of such factors.

In one embodiment, assays employing HEK293 cells transfected with an expressible TLR structural gene may use a threshold of, for example, at least a three-fold increase in a TLR-mediated biological activity (e.g., NFKB activation) when the compound is provided at a concentration of, for example, from about 1 µM to about 10 µM for identifying a compound as an agonist of the TLR transfected into the cell. However, different thresholds and/or different concentration ranges may be suitable in certain circumstances. Also, different thresholds may be appropriate for different assays.

Pretreatment of peripheral blood mononuclear cells (PBMCs) with a Type II interferon such as, for example, IFN-γ significantly increases the production of IL-12 by PBMCs stimulated with IRM compounds. In fact, IFN-γ priming has been shown to result in levels of IL-12 production comparable with those of healthy volunteers subjected to the same treatment. Thus, in certain embodiments, the methods of the invention can include contacting a cell population with a priming dose of a Type II IFN-γ or administering to a patient a priming dose of a Type II interferon. The Type II interferon may be recombinantly-derived or naturally-occurring.

### Methods

In one aspect, the invention includes a method of treating or ameliorating cutaneous T-cell lymphoma (CTCL) in a subject in need thereof. In certain non-limiting embodiments, the method comprises administering to the subject topically, transdermally, intradermally or intralesionally a therapeutically effective amount of a pharmaceutical composition comprising an immune response modifier (IRM) compound, whereby the CTCL in the subject is treated or ameliorated.

In another aspect, the invention includes a method of increasing a cell-mediated immune response in a subject suffering from CTCL. In certain non-limiting embodiments, the method comprises administering to the subject topically, transdermally, intradermally, or intralesionally a therapeutically effective amount of a pharmaceutical composition comprising an IRM compound, whereby the cell-mediated immune response in the subject is increased.

In various embodiments of any of the above aspects or any aspect of the invention delineated herein, the administration of the composition to the subject is topical. In certain embodiments of the invention, the IRM compound comprises 4-amino-**α,α**-dimethyl-2-ethoxymethyl-1H-imidazo[4,5-c]quinolin-1-ethanol or a pharmaceutically acceptable salt thereof. In other embodiments of the invention, the composition comprises a gel. In yet other embodiments of the invention, the composition comprises from about 0.01% (w/w) IRM to about 0.5% (w/w) IRM. In yet other embodiments of the invention, the composition comprises from about 0.03% (w/w) IRM to about 0.06% (w/w) IRM.

In various embodiments of any of the above aspects or any aspect of the invention delineated herein, the composition is applied to at least one CTCL lesion of the subject. In certain embodiments of the invention, the administration results in at least partial clearing of the at least one CTCL lesion to which the composition was applied. In other embodiments of the invention, the administration results in at least partial clearing of at least one CTCL lesion to which the composition was not applied. In yet other embodiments of the invention, the administration results in 50% or greater clearing of the total CTCL lesions in the subject. In yet other embodiments of the invention, the amount of the IRM administered to the subject is from about 1 µg/kg to about 10 mg/kg. In yet other embodiments of the invention, the amount of the IRM administered to the subject is from about 100 ng/lesion to about 1 mg/lesion. In yet other embodiments of the invention, the composition is administered to the subject at a frequency of at least once per day, at least once per week, or at least once per month. In yet other embodiments of the invention, the composition is administered to the subject repeatedly over a duration of at least one day, at least one week, at least one month, or at least one year.

In various embodiments of any of the above aspects or any aspect of the invention delineated herein, the administration activates a systemic cell-mediated antitumor immune response in the subject. In certain embodiments of the invention, the administration induces infiltration of activated NK cells or activated T-cells in at least one lesion in the subject. In other embodiments of the invention, the administration results in an increase level of granzyme or IFN **α** in at least one lesion in the subject. In yet other embodiments of the invention, the administration activates circulating myeloid dendritic cells or circulating NK cells in the blood of the subject. In yet other embodiments of the invention, the activated circulating myeloid dendritic cells have increased CD80 expression.

In various embodiments of any of the above aspects or any aspect of the invention delineated herein, the composition is administered to the subject during a first treatment period and during a second treatment period, and wherein the first treatment period and the second treatment period are separated by a non-treatment period. In certain embodiments of the invention, the composition is administered to the subject during the first treatment period at a frequency of at least once per day, at least once per week, or at least once per month. In other embodiments of the invention, the gel is administered to the subject during the second treatment period at a frequency of at least once per day, at least once per week, or at least once per month. In yet other embodiments of the invention, the first treatment period is at least about two weeks, at least about three weeks, at least about four weeks, at least about five weeks, at least about six weeks, at least about seven weeks, or at least about eight weeks. In yet other embodiments of the invention, the second treatment period is at least about two weeks, at least about three weeks, at least about four weeks, at least about five weeks, at least about six weeks, at least about seven weeks, or at least about eight weeks. In yet other embodiments of the invention, the non-treatment period separating the first treatment period and the second treatment period is at least about one week, at least about two weeks, at least about three weeks, or at least about four weeks. In yet other embodiments of the invention, the subject is a mammal. In yet other embodiments of the invention, the mammal is human.

### Formulation, Dosing and Administration

The IRM compound may be provided in a formulation suitable for contacting cells in vitro or for administration to a subject. In one embodiment, the formulation is administered to the subject by an inhalational, topical, oral, nasal, buccal, rectal, pleural, peritoneal, vaginal, intramuscular, subcutaneous, transdermal, epidural, intrathecal or intravenous route. In another embodiment, the formulation is administered topically, intradermally, transdermally or intralesionally. In yet another embodiment, the formulation containing the IRM compound is a gel.

In one embodiment, the subject is a bird or a mammal including but not limited to mouse, rat, ferret, guinea pig, non-human primate (such as monkey), dog, cat, horse, cow, pig and other farm animals. In another embodiment, the subject is a human.

In some embodiments, the methods of the present invention include administering an IRM compound to a subject in a formulation of, for example, from about 0.0001% to about 20% (unless otherwise indicated, all percentages provided herein are weight/weight with respect to the total formulation) to the subject, although in some embodiments the IRM compound may be administered using a formulation that provides IRM compound in a concentration outside of this range. In certain embodiments, the method includes administering to a subject a formulation that includes from about 0.01% to about 1 % IRM compound, for example, a formulation that includes about from about 0.1 % to about 0.5% IRM compound. In certain other embodiments, the methods include administering to a subject a formulation that includes from about 0.01% to about 0.5% IRM compound, for example, from about 0.01% to about 0.2% IRM compound, from about 0.01% to about 0.15% IRM compound, from about 0.01% to about 0.1% IRM compound, from about 0.025% to about 0.15% IRM compound, from about 0.025% to about 0.1% IRM compound, from about 0.05% to about 0.15% IRM compound, from about 0.05% to about 0.1% IRM compound, or about 0.01%, 0.02%, 0.03%, 0.04%, 0.05%, 0.06%, 0.07%, 0.08%, 0.09%, or 0.1% IRM compound.

An amount of an IRM compound effective for treating CTCL is an amount sufficient to limit, reduce, ameliorate, or slow the progression or severity of at least one symptom or clinical sign of CTCL. The precise amount of IRM compound for treating CTCL will vary according to factors known in the art including but not limited to the physical and chemical nature of the IRM compound, the nature of the carrier, the intended dosing regimen, the state of the subject's immune system (e.g., suppressed, compromised, stimulated), the method of administering the IRM compound, and the species to which the IRM compound is being administered. Accordingly, it is not practical to set forth specifically the amount that constitutes an amount of IRM compound effective for treating CTCL for all possible applications. Those of ordinary skill in the art, however, can readily determine the appropriate amount with due consideration of such factors.

In some embodiments, the methods of the present invention include administering sufficient IRM compound to provide a dose of, for example, from about 100 pg/kg to about 50 mg/kg to the subject, although in some embodiments the methods may be performed by administering IRM compound in a dose outside this range. In some of these embodiments, the method includes administering sufficient IRM compound to provide a dose of from about 10 ng/kg to about 10 mg/kg to the subject. In some embodiments, the dose may be calculated using actual body weight obtained just prior to the beginning of the treatment course.

Body surface area (m²) may be calculated prior to the beginning of the treatment course using the Dubois method: m² = (wt kg^{0.425} x height cm^{0,725}) x 0.007184. In such embodiments, methods of the present invention include administering sufficient IRM compound to provide a dose of, for example, from about 0.01 mg/m² to about 500 mg/m² to the patient, although in some embodiments the methods may be performed by administering IRM compound in a dose outside this range. In some of these embodiments, the method includes administering sufficient IRM to provide a dose of from about 0.1 mg/m² to about 250 mg/m² to the patient.

In some embodiments, the methods of the present invention include administering sufficient IRM compound to a lesion, to provide a dose per application of, for example, from about 100 ng/lesion to about 1 mg/lesion of the subject. In some of these embodiments, the method includes administering sufficient IRM compound to provide a dose per application of, for example, from about 100 µg/lesion to about 1 mg/lesion of the subject. In various embodiments, the method includes administering sufficient IRM compound to provide a dose per application of, for example, at least about 100 µg/lesion, at least about 200 µg/lesion, at least about 300 µg/lesion, at least about 400 µg/lesion, at least about 500 µg/lesion, at least about 600 µg/lesion, at least about 700 µg/lesion, at least about 800 µg/lesion, or at least about 900 µg/lesion of the subject.

The dosing regimen and duration of therapy may depend at least in part on many factors known in the art including but not limited to the physical and chemical nature of the IRM compound, the nature of the carrier, the amount of IRM being administered, the state of the subject's immune system (e.g., suppressed, compromised, stimulated), the method of administering the IRM compound, and the species to which the IRM compound is being administered. Accordingly it is not practical to set forth specifically the dosing regimen and duration of therapy effective for treating CTCL for all possible applications. Those of ordinary skill in the art, however, can readily determine an appropriate dosing regimen and therapy duration with due consideration of such factors.

In some embodiments, the IRM compound may be administered on an "as needed" basis, i.e., only when symptoms or clinical signs of CTCL appear. In other embodiments, the IRM compound may be administered over a prescribed duration of time, such as at least a day, at least a week, at least a month, or at least a year. In some embodiments of the invention, the IRM compound may be administered, for example, from a single administration to a frequency of at least once per day for an extended time. In some embodiments, the IRM compound may be administered about at least once per day, about at least once per week, or about at least once per month. In some embodiments, the IRM compound may be administered about at least twice per day, about at least twice per week, or about at least twice per month. Administration of the IRM compound may be continuous throughout a prescribed period of time or, alternatively, one or more rest periods may be incorporated into the prescribed period of time.

The duration of therapy may be, for example, at least one week, at least two weeks, at least three weeks, at least four weeks, at least five weeks, at least six weeks, at least seven weeks, at least eight weeks, at least three months, at least four months, at least five months, at least six months, at least seven months, at least eight months, at least nine months, at least ten months, at least eleven months, or at least one year.

In various embodiments, the IRM compound may be administered at least one, at least two, at least three, at least four, at least five, at least six, or at least seven times per week for at least one, at least two, at least three, at least four, at least five, at least six, at least seven, at least eight, at least nine, at least ten, at least eleven, at least twelve, at least thirteen, at least fourteen, at least fifteen, or at least sixteen weeks, in at least one, at least two, at least three, at least four, at least five, or at least six cycles having no rest period included or with having at least one, at least two, at least three, at least four, at least five, or at least six rest periods of at least one, at least two, at least three, at least four, at least five, at least six, at least seven, at least eight, at least nine, or at least ten weeks included. In particular embodiments, the IRM compound is administered at least one, at least two, at least three, at least four, at least five, at least six, or at least daily for each of two 8-week cycles having a 4-week rest period between each 8-week cycle.

Pharmaceutical compositions that are useful in the methods of the invention may be suitably developed for nasal, inhalational, oral, rectal, vaginal, pleural, peritoneal, parenteral, topical, transdermal, pulmonary, intranasal, buccal, ophthalmic, epidural, intrathecal, intravenous or another route of administration. In a preferred embodiment, the compositions are developed for topical, intradermal, transdermal or intralesional administration.

Routes of administration of any of the compositions of the invention include inhalational, oral, nasal, rectal, parenteral, sublingual, transdermal, transmucosal (e.g., sublingual, lingual, (trans)buccal, (trans)urethral, vaginal (e.g., trans- and perivaginally), (intra)nasal, and (trans)rectal), intravesical, intrapulmonary, intraduodenal, intragastrical, intrathecal, epidural, intrapleural, intraperitoneal, subcutaneous, intramuscular, intradermal, intra-arterial, intravenous, intrabronchial, inhalation, and topical administration.

Suitable compositions and dosage forms include, for example, tablets, capsules, caplets, pills, gel caps, troches, emulsions, dispersions, suspensions, solutions, syrups, granules, beads, transdermal patches, gels, powders, pellets, magmas, lozenges, creams, pastes, plasters, lotions, discs, suppositories, and liquid sprays. It should be understood that the formulations and compositions that would be useful in the present invention are not limited to the particular formulations and compositions that are described herein.

An obstacle for topical administration of pharmaceuticals is the stratum corneum layer of the epidermis. The stratum corneum is a highly resistant layer comprised of protein, cholesterol, sphingolipids, free fatty acids and various other lipids, and includes cornified and living cells. One of the factors that limit the penetration rate (flux) of a compound through the stratum corneum is the amount of the active substance that can be loaded or applied onto the skin surface. The greater the amount of active substance which is applied per unit of area of the skin, the greater the concentration gradient between the skin surface and the lower layers of the skin, and in turn the greater the diffusion force of the active substance through the skin. Therefore, a formulation containing a greater concentration of the active substance is more likely to result in penetration of the active substance through the skin, and more of it, and at a more consistent rate, than a formulation having a lesser concentration, all other things being equal.

Formulations suitable for topical administration include, but are not limited to, liquid or semi liquid preparations such as liniments, lotions, oil-in-water or water-in-oil emulsions such as creams, ointments or pastes, and solutions or suspensions. Topically administrable formulations may, for example, comprise from about 1% to about 10% (w/w) active ingredient, although the concentration of the active ingredient may be as high as the solubility limit of the active ingredient in the solvent. Formulations for topical administration may further comprise one or more of the additional ingredients described herein.

Enhancers of permeation may be used. These materials increase the rate of penetration of drugs across the skin. Typical enhancers in the art include ethanol, glycerol monolaurate, PGML (polyethylene glycol monolaurate), dimethylsulfoxide, and the like. Other enhancers include oleic acid, oleyl alcohol, ethoxydiglycol, laurocapram, alkanecarboxylic acids, dimethylsulfoxide, polar lipids, or N-methyl-2-pyrrolidone.

One acceptable vehicle for topical delivery of some of the compositions of the invention may contain liposomes. The composition of the liposomes and their use are known in the art, for example, U.S. Patent No. 6,323,219.

In alternative embodiments, the topically active pharmaceutical composition may be optionally combined with other ingredients such as adjuvants, anti-oxidants, chelating agents, surfactants, foaming agents, wetting agents, emulsifying agents, viscosifiers, buffering agents, preservatives, and the like. In another embodiment, a permeation or penetration enhancer is included in the composition and is effective in improving the percutaneous penetration of the active ingredient into and through the stratum corneum with respect to a composition lacking the permeation enhancer. Various permeation enhancers, including oleic acid, oleyl alcohol, ethoxydiglycol, laurocapram, alkanecarboxylic acids, dimethylsulfoxide, polar lipids, or N-methyl-2-pyrrolidone, are known to those of skill in the art. In another aspect, the composition may further comprise a hydrotropic agent, which functions to increase disorder in the structure of the stratum corneum, and thus allows increased transport across the stratum corneum. Various hydrotropic agents such as isopropyl alcohol, propylene glycol, or sodium xylene sulfonate, are known to those of skill in the art.

The topically active pharmaceutical composition should be applied in an amount effective to affect desired changes. As used herein "amount effective" shall mean an amount sufficient to cover the region of skin surface where a change is desired. An active compound should be present in the amount of from about 0.0001% to about 15% by weight volume of the composition. More preferable, it should be present in an amount from about 0.0005% to about 5% of the composition; most preferably, it should be present in an amount of from about 0.001 % to about 1% of the composition. Such compounds may be synthetically-or naturally derived.

Additional dosage forms of this invention include dosage forms as described in U.S. Patents Nos. 6,340,475, 6,488,962, 6,451,808, 5,972,389, 5,582,837, and 5,007,790. Additional dosage forms of this invention also include dosage forms as described in U.S. Patent Applications Nos. 20030147952, 20030104062, 20030104053, 20030044466, 20030039688, and 20020051820. Additional dosage forms of this invention also include dosage forms as described in PCT Applications Nos. WO 03/35041, WO 03/35040, WO 03/35029, WO 03/35177, WO 03/35039, WO 02/96404, WO 02/32416, WO 01/97783, WO 01/56544, WO 01/32217, WO 98/55107, WO 98/11879, WO 97/47285, WO 93/18755, and WO 90/11757.

### Controlled Release Formulations and Drug Delivery Systems

Controlled- or sustained-release formulations of a pharmaceutical composition of the invention may be made using conventional technology. In some cases, the dosage forms to be used can be provided as slow or controlled-release of one or more active ingredients therein using, for example, hydropropylmethyl cellulose, other polymer matrices, gels, permeable membranes, osmotic systems, multilayer coatings, microparticles, liposomes, or microspheres or a combination thereof to provide the desired release profile in varying proportions. Suitable controlled-release formulations known to those of ordinary skill in the art, including those described herein, can be readily selected for use with the pharmaceutical compositions of the invention.

Most controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood level of the drug, and thus can affect the occurrence of side effects.

Most controlled-release formulations are designed to initially release an amount of drug that promptly produces the desired therapeutic effect, and gradually and continually release of other amounts of drug to maintain this level of therapeutic effect over an extended period of time. In order to maintain this constant level of drug in the body, the drug must be released from the dosage form at a rate that will replace the amount of drug being metabolized and excreted from the body.

Controlled-release of an active ingredient can be stimulated by various inducers, for example pH, temperature, enzymes, water, or other physiological conditions or compounds. The term "controlled-release component" in the context of the present invention is defined herein as a compound or compounds, including, but not limited to, polymers, polymer matrices, gels, permeable membranes, liposomes, or microspheres or a combination thereof that facilitates the controlled-release of the active ingredient.

In certain embodiments, the formulations of the present invention may be, but are not limited to, short-term, rapid-offset, as well as controlled, for example, sustained release, delayed release and pulsatile release formulations.

The term sustained release is used in its conventional sense to refer to a drug formulation that provides for gradual release of a drug over an extended period of time, and that may, although not necessarily, result in substantially constant blood levels of a drug over an extended time period. The period of time may be as long as a month or more and should be a release that is longer that the same amount of agent administered in bolus form.

For sustained release, the compounds may be formulated with a suitable polymer or hydrophobic material which provides sustained release properties to the compounds. As such, the compounds for use the method of the invention may be administered in the form of microparticles, for example, by injection or in the form of wafers or discs by implantation.

In a preferred embodiment of the invention, the compounds of the invention are administered to a patient, alone or in combination with another pharmaceutical agent, using a sustained release formulation.

The term delayed release is used herein in its conventional sense to refer to a drug formulation that provides for an initial release of the drug after some delay following drug administration and that may, although not necessarily, includes a delay of from about 10 minutes up to about 24 hours.

The term pulsatile release is used herein in its conventional sense to refer to a drug formulation that provides release of the drug in such a way as to produce pulsed plasma profiles of the drug after drug administration.

The term immediate release is used in its conventional sense to refer to a drug formulation that provides for release of the drug immediately after drug administration.

As used herein, short-term refers to any period of time up to and including about 24 hours, about 12 hours, about 8 hours, about 7 hours, about 6 hours, about 5 hours, about 4 hours, about 3 hours, about 2 hours, about 1 hour, about 40 minutes, about 20 minutes, or about 10 minutes and any or all whole or partial increments thereof after drug administration after drug administration.

As used herein, rapid-offset refers to any period of time up to and including about 24 hours, about 12 hours, about 8 hours, about 7 hours, about 6 hours, about 5 hours, about 4 hours, about 3 hours, about 2 hours, about 1 hour, about 40 minutes, about 20 minutes, or about 10 minutes, and any and all whole or partial increments thereof after drug administration.

Those skilled in the art will recognize, or be able to ascertain using no more than routine experimentation, numerous equivalents to the specific procedures, embodiments, claims, and examples described herein. Such equivalents were considered to be within the scope of this invention and covered by the claims appended hereto. For example, it should be understood, that modifications in reaction conditions, including but not limited to reaction times, reaction size/volume, and experimental reagents, such as solvents, catalysts, pressures, atmospheric conditions, e.g., nitrogen atmosphere, and reducing/oxidizing agents, with art-recognized alternatives and using no more than routine experimentation, are within the scope of the present application.

It is to be understood that wherever values and ranges are provided herein, all values and ranges encompassed by these values and ranges, are meant to be encompassed within the scope of the present invention. Moreover, all values that fall within these ranges, as well as the upper or lower limits of a range of values, are also contemplated by the present application.

The following examples further illustrate aspects of the present invention. However, they are in no way a limitation of the teachings or disclosure of the present invention as set forth herein.

### EXAMPLES

The invention is now described with reference to the following Examples. These Examples are provided for the purpose of illustration only, and the invention is not limited to these Examples, but rather encompasses all variations that are evident as a result of the teachings provided herein.

### Example 1:

As described herein for the first time, a Phase I open label trial of varying doses of resiquimod gel for stages IA-IIA CTCL patients was initiated.

Four highly refractory patients (three on stage IB; one on stage IIA), who had failed more than six previous treatments on average, including interferon, oral bexarotene, PUVA (psoralen + UVA radiation), topical nitrogen mustard, topical carmustine, potent topical steroids and electron beam radiation, applied no more than 500 mg/application of 0.06% resiquimod gel to up to four target lesions at varying frequencies for two eight-week cycles with a four-week rest period in between.

All treated patients have experienced clearing of treated target lesions, as well as improvement in non-treated distant lesions and improvement in severity of pruritus.

All patients experienced several days of low grade fever upon initiation of the drug. Preliminary results demonstrate a marked intralesional influx of activated cytotoxic T-cells and NK cells manifesting increased expression of granzyme and interferon gamma. Evidence for systemic immune augmentation includes progressive activation of circulating myeloid dendritic cells and NK cells.

The Phase I results indicate that the resiquimod gel is an effective therapeutic agent for CTCL with potent immune augmenting properties. The results reported herein indicate that resiquimod induces high clinical response rates of both treated as well as distant, non-treated lesions, and possesses the ability to significantly boost systemic cellular immunity directed against CTCL. The importance of these findings is significant not only for treatment of CTCL, but for treatment of other skin cancers as well.

### Example 2.

The Phase I trial reported in Example 1 was expanded to a total of ten patients (all of which had failed at least two prior therapies, and most of which had failed five or more prior therapies). Eight of those patients received treatment with 0.06% resiquimod gel, while the two remaining patients received treatment with 0.03% resiquimod gel.

The final trial design calls for a total of 16-20 patients, with the first half cohort treated topically with 0.06% gel and the second half cohort treated topically with 0.03% gel.

According to the study design, treatment of only four to five target lesions is permitted for eight weeks; followed by four weeks of rest and another eight weeks of therapy. Final evaluation is conducted after four weeks off of therapy.

Initial data was made available for nine patients.

Eight of those nine patients have shown a significant response to treatment as measured by lesion reduction. Out of those eight patients, all have had a significant clinical response (defined as more than 50% decrease in extent of skin involvement by skin lesions of cutaneous T-cell lymphoma), and two have had experienced a complete clinical response (defined as clearing of all evidence of disease in skin and elsewhere). Figure 1 comprises photographs of one patient in the Phase I study. This patient, which was refractory to previous treatments, showed clearance of skin lesions by week 8 of therapy.

This is an unexpectedly high rate of response, which would be considered in the field as unprecedented since most topical agents produce response rates of about 50% or less. The present treatment has been well tolerated without any serious adverse effects; the only minor problems have been some skin irritation cause by the application of the gel.

Analysis of the trial results indicated that there was marked activation of anti-tumor immune responses directly at lesional sites treated with the topical gel. This response included infiltration of the lesions during therapy by highly activated natural killer cells as well as activated CD8+ cytotoxic T-cells. Both NK cells and CD8+ T-cells entering lesions during therapy expressed high levels of interferon gamma, granzyme and perforin (wherein granzyme and perforin are cytolytic enzymes necessary for killing of the tumor cells).

Analysis of the trial results also indicated activation of important immune cells in the peripheral blood of the patients, including myeloid dendritic cells (as evidenced by increased expression of the co-stimulatory molecule CD80) and circulating natural killer cells (as evidenced by increased expression of CD107 on the CD56+ NK cells).

Figures 2 illustrates increased CD80 expression on CD11c+ myeloid dendritic cells in the peripheral blood of patients, indicating activation of these cells during the initial eight weeks of therapy and again after the four- week treatment hiatus ending week 12.

The results obtained so far are unprecedented, in view of the prior less promising data on cutaneous treatment of CTCL lesions in actual clinical practice. The resiquimod trial for CTCL demonstrated that the topical treatment is well-tolerated, easy to comply with, and shows only grade I skin toxicity. The clinical response rates were high for both treated and untreated lesions among refractory early stage CTCL patients. Further, the studies demonstrated systemic immune activation in the patients treated with the resiquimod gel.

The disclosures of each and every patent, patent application, and publication cited herein are hereby incorporated herein by reference in their entirety. While this invention has been disclosed with reference to specific embodiments, it is apparent that other embodiments and variations of this invention may be devised by others skilled in the art without departing from the true spirit and scope of the invention. The appended claims are intended to be construed to include all such embodiments and equivalent variations.

The invention is further embodied by the following items:
1. A method of treating or ameliorating cutaneous T-cell lymphoma (CTCL) in a subject in need thereof, the method comprising administering to the subject topically, transdermally, intradermally or intralesionally a therapeutically effective amount of a pharmaceutical composition comprising an immune response modifier (IRM) compound, whereby the CTCL in the subject is treated or ameliorated.
2. The method of item 1, wherein the administration of the composition to the subject is topical.
3. The method of item 1, wherein the IRM compound comprises 4-amino-**α,α-**dimethyl-2-ethoxymethyl-1H-imidazo[4,5-c]quinolin-1-ethanol or a pharmaceutically acceptable salt thereof.
4. The method of item 1, wherein the composition comprises a gel.
5. The method of item 1, wherein the composition comprises from about 0.01% (w/w) IRM to about 0.5% (w/w) IRM.
6. The method of item 5, wherein the composition comprises from about 0.03% (w/w) IRM to about 0.06% (w/w) IRM.
7. The method of item 1, wherein the composition is applied to at least one CTCL lesion of the subject.
8. The method of item 7, wherein the administration results in at least partial clearing of the at least one CTCL lesion to which the composition was applied.
9. The method of item 7, wherein the administration results in at least partial clearing of at least one CTCL lesion to which the composition was not applied.
10. The method of item 1, wherein the administration results in 50% or greater clearing of the total CTCL lesions in the subject.
11. The method of item 1, wherein the amount of the IRM administered to the subject is from about 1 µg/kg to about 10 mg/kg.
12. The method of item 11, wherein the amount of the IRM administered to the subject is from about 100 ng/lesion to about 1 mg/lesion.
13. The method of item 1, wherein the composition is administered to the subject at a frequency of at least once per day, at least once per week, or at least once per month.
14. The method of item 1, wherein the composition is administered to the subject repeatedly over a duration of at least one day, at least one week, at least one month, or at least one year.
15. The method of item 1, wherein the administration activates a systemic cell-mediated antitumor immune response in the subject.
16. The method of item 15, wherein the administration induces infiltration of activated NK cells or activated T-cells in at least one lesion in the subject.
17. The method of item 16, wherein the administration results in an increase level of granzyme or IFN-**α** in at least one lesion in the subject.
18. The method of item 15, wherein the administration activates circulating myeloid dendritic cells or circulating NK cells in the blood of the subject.
19. The method of item 18, wherein the activated circulating myeloid dendritic cells have increased CD80 expression.
20. The method of item 1, wherein the composition is administered to the subject during a first treatment period and during a second treatment period, and wherein the first treatment period and the second treatment period are separated by a non-treatment period.
21. The method of item 20, wherein the composition is administered to the subject during the first treatment period at a frequency of at least once per day, at least once per week, or at least once per month.
22. The method of item 20, wherein the gel is administered to the subject during the second treatment period at a frequency of at least once per day, at least once per week, or at least once per month.
23. The method of item 20, wherein the first treatment period is at least about two weeks, at least about three weeks, at least about four weeks, at least about five weeks, at least about six weeks, at least about seven weeks, or at least about eight weeks.
24. The method of item 20, wherein the second treatment period is at least about two weeks, at least about three weeks, at least about four weeks, at least about five weeks, at least about six weeks, at least about seven weeks, or at least about eight weeks.
25. The method of item 20, wherein the non-treatment period separating the first treatment period and the second treatment period is at least about one week, at least about two weeks, at least about three weeks, or at least about four weeks.
26. The method of item 1, wherein the subject is a mammal.
27. The method of item 26, wherein the mammal is human.
28. A method of increasing a cell-mediated immune response in a subject suffering from CTCL, the method comprising administering to the subject topically, transdermally, intradermally, or intralesionally a therapeutically effective amount of a pharmaceutical composition comprising an immune response modifier (IRM) compound, whereby the cell-mediated immune response in the subject is increased.
29. The method of item 28, wherein the IRM compound comprises 4-amino-**α,α**-dimethyl-2-ethoxymethyl-1H-imidazo[4,5-c]quinolin-1-ethanol or a pharmaceutically acceptable salt thereof.
30. The method of item 28, wherein the composition comprises from about 0.01% (w/w) IRM to about 0.5% (w/w) IRM.
31. The method of item 28, wherein the composition is applied to at least one CTCL lesion of the subject.
32. The method of item 31, wherein the administration results in at least partial clearing of at least one selected from the group consisting of a CTCL lesion to which the composition was applied or a CTCL lesion to which the composition was not applied.
33. The method of item 28, wherein the composition is administered to the subject at a frequency of at least once per day, at least once per week, or at least once per month.
34. The method of item 28, wherein the composition is administered to the subject during a first treatment period and during a second treatment period, and wherein the first treatment period and the second treatment period are separated by a non-treatment period.
35. The method of item 28, wherein the subject is a mammal.
36. The method of item 35, wherein the mammal is human.

## Claims

1. A composition comprising a TLR 7/8 agonist immune response modulator (IRM) compound, or a pharmaceutically acceptable salt thereof, for use in treating or ameliorating T-cell lymphoma (CTCL) in a subject suffering from CTCL,
wherein the composition is to be administered topically, transdermally, intradermally, or intralesionally, and
wherein the administration results in at least partial clearing of at least one CTCL lesion to which the composition was not applied.

2. The composition of claim 1, wherein the TLR 7/8 agonist is 4-amino-α,α-dimethyl-2-ethoxymethyl-1H-imidazo[4,5-c]quinolin-1-ethanol (resiquimod).

3. The composition for use of any of claims 1-2, wherein the composition is applied to at least one CTCL lesion of the subject.

4. The composition for use of any of claims 1-3, wherein the administration results in 50% or greater clearing of the total CTCL lesions in the subject.

5. The composition for use of any of claims 1-4, wherein the amount of the TLR 7/8 agonist administered to the subject is from about 1 µg/kg to about 10 mg/kg.

6. The composition for use of any of claims 1-5, wherein the composition is administered to the subject at a frequency of at least once per day, at least once per week, or at least once per month.

7. The composition for use of any of claims 1-6, wherein the compound is administered to the subject during a first treatment period and during a second treatment period, and wherein the first treatment period and the second treatment period are separated by a non-treatment period.

8. The composition for use of claim 7, wherein the composition is administered to the subject during the first treatment period at a frequency of at least once per day, at least once per week, or at least once per month.

9. The composition for use of claim 7, wherein the first treatment period is at least about two weeks, at least about three weeks, at least about four weeks, at least about five weeks, at least about six weeks, at least about seven weeks, or at least about eight weeks.

10. The composition for use of claim 7, wherein the second treatment period is at least about two weeks, at least about three weeks, at least about four weeks, at least about five weeks, at least about six weeks, at least about seven weeks, or at least about eight weeks.

11. The composition for use of claim 7, wherein the non-treatment period separating the first treatment period and the second treatment period is at least about one week, at least about two weeks, at least about three weeks, or at least about four weeks.

12. The composition for use of any of claims 1-11, wherein the subject is a human.

13. The composition for use of any of claims 1-12, wherein the composition comprises a gel.

14. The composition for use of any of claims 1-13, wherein the administration results in at least partial clearing of at least one CTCL lesion to which the composition was applied.

15. The composition for use of claim 2, wherein the composition comprises about 0.03-0.06% (w/w) of resiquimod.
